(19) Europäisches Patentamt
European Patent Office
Office européen des brevets

(11) EP 1 122 044 B1

(12) EUROPEAN PATENT SPECIFICATION

(45) Date of publication and mention of the grant of the patent:
**20.04.2005 Bulletin 2005/16**

(51) Int Cl.$^7$: **B27K 3/50**, B27K 3/34, C07C 69/52, C07C 69/28, A01N 33/12, C11D 1/74

(21) Application number: **01101120.2**

(22) Date of filing: **09.06.1994**

(54) **Use of waterproofer composition for the treatment of wood**

Verwendung einer Wasserdichtungszusammensetzung für die Konservierung von Holz

Utilisation d'une composition imperméabilisante pour la traitement du bois

(84) Designated Contracting States:
**AT BE CH DE DK ES FR GB GR IE IT LI LU MC NL PT SE**
Designated Extension States:
**SI**

(30) Priority: **09.06.1993 US 74136**
**09.06.1993 US 74312**
**09.06.1993 US 74313**
**09.06.1993 US 74314**

(43) Date of publication of application:
**08.08.2001 Bulletin 2001/32**

(62) Document number(s) of the earlier application(s) in accordance with Art. 76 EPC:
**94920194.1 / 0 702 517**

(73) Proprietor: **LONZA INC.**
**Fair Lawn New Jersey 07410 (US)**

(72) Inventor: **Walker, Leigh**
**Macungie, PA 18062 (US)**

(74) Representative: **Riegler, Norbert Hermann**
**Lonza AG**
**Patentabteilung**
**Postfach**
**4002 Basel (CH)**

(56) References cited:
**EP-A- 0 342 609**
**WO-A-87/06177**
**DE-A- 3 519 557**
**DE-B- 2 916 304**
**US-A- 3 888 728**
**US-A- 4 732 817**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

### FIELD OF THE INVENTION

This invention relates to waterproofing and wood preservation compositions. Polyhydroxyl or polyether hydroxyl esters of fatty acids and polyether hydroxides have been found to be useful as waterproofers for wood substrates. Furthermore, these waterproofers in combination with quaternary ammonium compositions and a solvent are useful as waterproofing wood preservation compositions. Preferred quaternary ammonium compositions include $C_1$-$C_{20}$ alkyl or aryl-substituted alkyl, $C_8$-$C_{20}$ alkyl quaternary ammonium chlorides, hydroxides, carbonates, carboxylates, or borates.

### BACKGROUND OF THE INVENTION

Quaternary ammonium compounds (quats), and particularly didecyldimethylammonium chloride (DDAC)

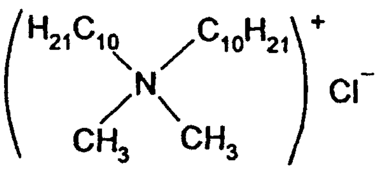

(I)

are commonly used as wood preservatives because they possess resistance properties to fungi and termites, to loss of strength, and to electrical sensitivity similar to those of commonly used acidic copper/chromium/arsenic solution (CCA) or ammoniacal copper and arsenic salt solution preservatives. See Proc.of the Am.Wood Pres.Assoc. 80: 191-210 (1984). Although chloride quats do not include potentially dangerous heavy metals, didecyldimethylammonium chloride leaches rapidly in soil (Nicholas et al., Forest Prod. J., 41:41 (1991), and therefore, does require coupling with copper salt.

Findlay et al., US Patent No. 4,929,454, disclose a method, of preserving wood by impregnation with a quaternary ammonium compound and at least one of zinc and copper, wherein the quat anion is chosen from the group consisting of hydroxide, chloride, bromide, nitrate, bisulfate, acetate, bicarbonate, and carbonate, formate, borate and fatty acids. These quats have distinct environmental and safety advantages over commonly used acidic copper/chromium/arsenic solution (CCA) or ammoniacal copper and arsenic salt solution preservatives in that potentially dangerous heavy metals are not included. The Findlay et al. quats require copper or zinc in order to render them relatively insoluble and to prevent them from leaching out of a treated substrate. The use of copper or zinc in the above formulations may yet raise environmental and corrosion concerns.

Additionally, didecyldimethylammonium chloride tends to absorb preferentially to the surface of the wood and does not uniformly treat the whole substrate. Finally, DDAC treated wood shows a surface erosion or ages upon exposure to light. See Preston et al., Proc. Am. Wood Pres. Assoc., 83:331 (1987).

The biocidal activities of various chloride quats against bacteria, fungi, and algae are tabulated in Cationic Surfactants, E. Jungerman Ed., pp. 56-57, Marcel Dekker, Inc., 1969. Nicholas, "Interaction of Preservatives with Wood," Chemistry of Solid Wood, Advances in Chemistry Series #207, Powell ed., (A.C.S. 1984), notes that didecyldimethylammonium compounds and particularly DDAC are potential biocides. Preston, J.A.O.C.S. 60:567 (1983), concurs and suggests that maximum fungitoxicity is exhibited with dialkyldimethyl compounds having $C_{10}$-$C_{12}$ alkyl groups. Butcher et al., Chem.Abstracts No. 91:152627b, suggest that the presence of an acid or a base can affect the activity of didecyldimethylammonium quats.

Didecyldimethylammonium acetate was used as a phase transfer catalyst for an oxidation in Chem Abstracts No. 97:9175. A wood preservative was prepared by autoclaving didecylmethylamine with gluconic acid and ethylene oxide in isopropanol to yield $[(C_{10}H_{21})_2CH_3N((CH_2)_2O)_nH]^+$ gluconate in Chem. Abstracts No. 109:124403x, while disinfectant solutions were prepared by exchanging a benzylammonium chloride with a chlorhexidine gluconate in Chem.Abstracts No. 103:109954f.

Microbiocidal compositions which include quaternary ammonium compounds of the formula $R^1N^+R^2R^3R^4\ X^-$, wherein at least one of $R^1$, $R^2$, or $R^3$ is a $C_8$-$C_{30}$ alkyl or alkenyl group and the remainder of $R^1$, $R^2$ or $R^3$ is methyl, ethyl, $CH_2Ph$ or 4-pyridylmethyl; $R^4$ is methyl or ethyl; and X is an anion of an acid having a $C_7$ or greater hydrophobic group, were disclosed in Chem. Abstracts Nos. 113:154360f and 113:153776j. Chem. Abstracts No. 112:79768u discloses compounds of the formula $R^1R^2R^3R^4N^+X^-$, wherein $R^1$, $R^2$, and $R^3$ are methyl, ethyl, benzyl, 4-pyridinomethyl and at least one is $C_8$-$C_{30}$ alkyl or alkenyl; $R^4$ is methyl or ethyl; and X is a counter anion of acids having $C_7$ or greater

hydrophobic groups. Dimethyldidecylammonium dodecylbenzenesulfonate was demonstrated to impart long term rot resistance to wood without causing rust, while the chloride salts of similar compounds were demonstrated to cause rust.

Patton et al., U.S. Patent No. 5,004,760, disclose polymeric foams incorporating various dialkyldimethylammonium carboxylates such as didecyldimethylammonium poly (ethylene/acetate) and the like.

Quaternary ammonium compounds (quats) are typically prepared by the reaction:

$$R^1R^2R^3N + R^4X \rightarrow R^1R^2R^3R^4NX \qquad (II)$$

wherein X is a halogen, a sulface, a sulfo compound, or the like. When at least one of $R^1$, $R^2$, $R^3$, or $R^4$ is $C_{12}$ or longer, the product is an inert soap. Many of the inert soaps have biocidal activity against bacteria, fungi, algae, and related organisms.

Reaction (II) above is limited by the reactant $R^4X$ because $R^4$ must react with tertiary amines. For example, methyl chloride ($R^4X = CH_3Cl$) will react with a tertiary amine at less than 100°C to yield a quaternary compound $R_3N^+CH_3\ Cl^-$, while methanol or methyl acetate :$R^4X$-$CH_3OH$ or $CH_3COOCH_3$) will not, under similar reaction conditions.

General quaternary ammonium compounds with a sulfo group are easily prepared either by the reaction of a sulfonate compound with a tertiary amine (III) or by a double exchange (IV).

$$R_3N + RSO_3CH_3 \rightarrow R_3NCH_3{}^+RSO_3{}^- \qquad (III)$$

$$R_3N^+\ CH_3\ Cl^- + RSO_3{}^-\ Na^+ \rightarrow R_3NCH_3{}^+\ RSO_3{}^- + NaCl \qquad (IV)$$

If trimethylamine is heated with carbon dioxide and methanol above 200°C and at 85 to 95 atmospheres, the carbonate quat, bis-tetramethylammonium carbonate, is prepared. <u>Industrial Organic Nitrogen Compounds</u>, Astle Ed. p 66, Reinhold Inc, 1961. However, this reaction is limited to the methyl compound because higher homologs decompose to olefins by the Hofmann elimination reaction. See, <u>Organic Reactions</u>, 11, Chptr. 5, 377, Krieger Publishing Co., 1975.

Chem. Abstracts 110:212114 (1989) suggests that dimethyl carbonate will react with triethylamine in methanol in twelve hours at 115°C. and under pressure to yield a methyl carbonate ester quat.

Chem. Abstracts 114:24824 (1991) discloses that 6-hydroxyhexyldimethylamine reacts with dimethyl carbonate to yield a carbonate ester quat.

Quaternary ammonium hydroxides (hydroxy quats), an intermediate in the reaction scheme of the present invention, are currently prepared by the reaction of quaternary ammonium iodide with silver hydroxide (V).

$$RN^+(CH_3)_3\ I^- + AgOH \rightarrow RN^+(CH_3)_3OH^- + AgI \qquad (V)$$

However, this reaction is costly, and it is difficult to recover the silver reagent. See, <u>Organic Reactions</u>, 11:Chptr. 5, pp. 376-377, Krieger Publishing Co., 1975.

In an olefin synthesis, it has been suggested to treat a quaternary salt with aqueous sodium or potassium hydroxide followed by pyrolysis in order to form the hydroxy quat and then to decompose the hydroxy quat directly. However, in this method the hydroxy quat is not isolated and the conditions for its preparation are undesirable. See, <u>Organic Reactions,</u> 11:Chptr.5, pp. 376-377, Krieger Publishing Co., 1975.

Talmon et al., <u>Science</u>, 221, 1047 (1983), have used an ion exchange resin to convert didecyldimethylammonium bromide to didecyldimethylammonium hydroxide (VI).

$$(C_{12}H_{25})_2(CH_3)_2N^+\ Br^- + \text{Ion Exchange Resin} \rightarrow$$

$$(C_{12}H_{25})_2(CH_3)_2N^+OH^- \qquad (VI)$$

However, 50 ml of ion exchange resin and two treatment steps were required to convert 3 grams of quaternary ammonium chloride to the corresponding hydroxide. Talmon et al. state that the hydroxy quat can be reacted with acids to make quats with different anions, and they have prepared didodecyldimethylammonium (DDDA) acetate, DDDA-formate, DDDA-propionate, DDDA-butyrate, DDDA-oxalate, DDDA-acrylate, DDDA-tartrate, DDDA-benzoate, and DDDA-octanoate. See also, Organic Synthesis, Collective Volume VI, 552, John Wiley Inc., 1988; Brady et al., J. Am. Chem. Soc., 106:4280-4282, 1984; Brady et al., J. Phys. Chem., 90:9, 1853-1859, 1986; Miller et al., J. Phys. Chem, 91:1, 323-325, 1989; Radlinske et al., Colloids and Surfaces, 46:213-230, 1990.

Distearyldimethylammonium gluconate was prepared via ion exchange and subsequent reaction with an organic acid in Chem. Abstracts No. 75:11917OU. Miller et al., Langmuir, 4:1363 (1988) prepared ditetradecyldimethylammonium acetate by ion exchange from a bromide.

Alternatively, quaternary ammonium hydroxide compositions have been prepared by treating a haloquat in an electrochemical cell with special cation exchange diaphragms between the cells. The hydroxy quat collects at one electrode, and the halide collects at the other. Hydroxy quats, $R^1R^2R^3R^4N^+OH^-$, wherein the R groups were $C_1$-$C_4$, were treated with carboxylic acids to make asymmetric quats that were used as capacitor driving electrolytes. See, Japanese Patent Publication No. 02-106,915 and Awata et al., Chemistry, Letters, 371 (1985). Awata et al. placed carboxylic acids in the cathode cell to react with tetraethylammonium hydroxide as it was formed.

Japanese Patent Publication No. 01-172,363 discloses the preparation of relatively low yields of tetraethylammonium hydroxide by reacting triethylamine with diethyl sulfate, heating the resultant quat with sulfuric acid to yield the sulfate quat, and reacting the sulfate quat with barium hydroxide to yield the short chain quat, tetraethylammonium hydroxide, and barium sulfate.

Di $C_8$-$C_{12}$ alkyl quaternary ammonium hydroxides prepared by ion exchange were used as strong bases to digest animal tissue by Bush et al., French Patent Publication No. 1,518,427.

Akzo discloses that the addition of a metallic hydroxide to a quaternary ammonium chloride such as didecyldimethylammonium chloride, in an aqueous medium, results in an equilibrium mixture of quaternary ammonium chloride and quaternary ammonium hydroxide (VII). This reaction can be driven to the right by the use of isopropanol as a solvent.

$$(R_4N)Cl + KOH \rightarrow (R_4N)OH + KCl \qquad (VII)$$

Akzo further discloses that the addition of a soap to a quaternary ammonium chloride yields a quaternary ammonium carboxylate (VIII).

$$(R_4N)Cl + R^1COONa \rightarrow (R_4N)\,(OOCR^1) + NaCl \qquad (VIII)$$

Jordan et al., U.S. Patent No. 3,281,458, disclose the preparation of dioctadecyldimethylammonium humate, ditallowdimethylammonium humate, dipentadecyldimethylammonium humate, and didodecyldimethylammonium humate by reacting humic acid, lignite, aqueous sodium hydroxide and a chloride quat.

Finally, Nakama et al., J.A.C.O.S., 67:717 (1990) report the interaction between anionic and cationic surfactant and particularly sodium laurate and stearyltrimethylammonium chloride, while Linderborg, U.S. Patent No. 4,585,795, disclose the use of synergistic mixtures of the alkali metal salt of certain biocidal organic acids, quaternary ammonium chlorides, and alkyl-pyridinium chlorides as control agents for short-term protection of timber against sapstain fungi and mildew.

Typically, quaternary ammonium compounds migrate or leach from wood under wet conditions, however. Common waterproofing compositions have not proven compatible with the quaternary ammonium compounds typically used in the industry, and therefore, they are not commonly used to hinder the leaching of these quats.

Typical waterproofers are waxes, lower molecular weight polyolefins, or dispersions or solutions thereof in hydrocarbon solvents. However, quaternary compositions, including those useful in the present invention, typically are water soluble. Generally, they are not soluble in these typical waterproofer solvent systems and are not compatible with emulsified or dispersed waterproofers.

It has now been discovered that $C_1$-$C_{20}$ alkyl or aryl-substituted alkyl, $C_8$-$C_{20}$ alkyl, and particularly di $C_8$-$C_{12}$ alkyl, quaternary ammonium hydroxides, carbonates, carboxylates, and borates including those prepared by the methods described herein, are compatible with newly discovered polyhydroxyl or polyetherhydroxyl esters of fatty acids or polyether hydroxide waterproofers. Waterproofing and wood preservative systems prepared from the waterproofers or waterproofers and quats described herein exhibit enhanced resistance to leaching and meet waterproofing standards for heavy duty, ground, or millwork applications.

## BRIEF DESCRIPTION OF THE DRAWINGS

Figure 1A is a graphic comparison of leaching of waterproofer containing wood preservative systems according to the present invention and wood preservative systems without waterproofer.
Figure 1B is an enlarged section of Figure 1A.

According to the present invention there is provided use of a waterproofer composition for the treatment and preservation of wood, wherein said waterproofer composition comprises

(a) a compound having the formula

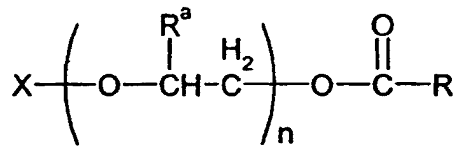

wherein:

X is hydrogen or

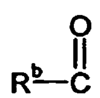 ,

R and $R^b$ independently are a (saturated or unsaturated) $C_9$-$C_{50}$ group,
$R^a$ is hydrogen or a methyl group; and
n is an integer from 1 to 10;

(b) a biocidal effective amount of at least one $C_1$-$C_{20}$ alkyl or aryl-substituted alkyl, $C_8$-$C_{20}$ alkyl quaternary ammonium composition; and
(c) optionally, a solvent,

said composition being metal-free.

In further embodiments, methods for waterproofing or waterproofing and preserving a wood substrate are provided wherein the substrate is treated with the waterproofer or waterproofer preservative systems above.

Quaternary Ammonium Hydroxide

Although any quaternary ammonium hydroxides are suitable for use in the present invention, quaternary ammonium hydroxides (hydroxy quats) having the formula

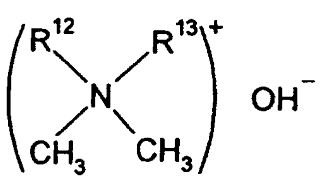

(XVI)

wherein $R^{12}$ is a $C_1$-$C_{20}$ alkyl or aryl-substituted alkyl group, $R^{13}$ is a $C_8$-$C_{20}$ alkyl group, and preferably $R^{12}$ is the same as $R^{13}$ and $R^{12}$ is a $C_8$-$C_{12}$ alkyl group, are preferred.

Special mention is made of hydroxy quats wherein $R^{12}$ is a methyl, $C_8$ alkyl, $C_9$ isoalkyl, $C_{10}$ alkyl, $C_{12}$ alkyl,

$C_{14}$ alkyl, $C_{16}$ alkyl, or benzyl group; and $R^{13}$ is a $C_{10}$ alkyl, $C_{12}$, $C_{14}$ alkyl or $C_{16}$ alkyl group. Most preferred hydroxy quats are didecyldimethylammonium hydroxide wherein $R^{12}$ and $R^{13}$ are a $C_{10}$ alkyl group and most preferably an n-$C_{10}$ group.

Didecyldimethylammonium hydroxide, when observed in a 70 to 80 percent by weight solution in a 50 percent by weight alcohol/50 percent by weight water solvent, is a yellow/orange liquid. This formulation has a flash point of about 134°F, and it is a highly alkaline material that reacts with the phenolic OH of lignin.

Quaternary ammonium hydroxides useful in the present invention are preferably prepared according to the reaction illustrated below in scheme XXV.

The method provides increased yields of $C_1$-$C_{20}$ alkyl or aryl-substituted alkyl, $C_8$-$C_{20}$ alkyl quaternary ammonium hydroxide, and preferably di $C_8$-$C_{12}$ alkyl quaternary ammonium hydroxide, when compared with conventional production methods. Although it was previously believed that the reaction of the chloride quat salt with a metal hydroxide to yield quaternary ammonium hydroxide and metal chloride was an equilibrium reaction (VII) or could be driven to the right by the use of branched solvents, it has now been discovered that by selection of the proper reactants, reaction medium, and/or reaction conditions (including reactant amounts), the reaction can be driven well past equilibrium to yield unprecedented greater amounts of $C_1$-$C_{20}$ alkyl or aryl-substituted alkyl, $C_8$-$C_{20}$ alkyl quaternary ammonium hydroxide.

Although the present method can be used to prepare a variety of $C_1$-$C_{20}$ alkyl or aryl-substituted alkyl, $C_8$-$C_{20}$ alkyl quaternary ammonium hydroxide compounds, the preferred reaction product quat is a di $C_8$-$C_{12}$ alkyl quaternary ammonium hydroxide compound. Most preferred hydroxy quats are di n-$C_8$-$C_{12}$ alkyl quaternary ammonium hydroxide, didecyldimethylammonium hydroxide, and di-n-decyldimethylammonium hydroxide.

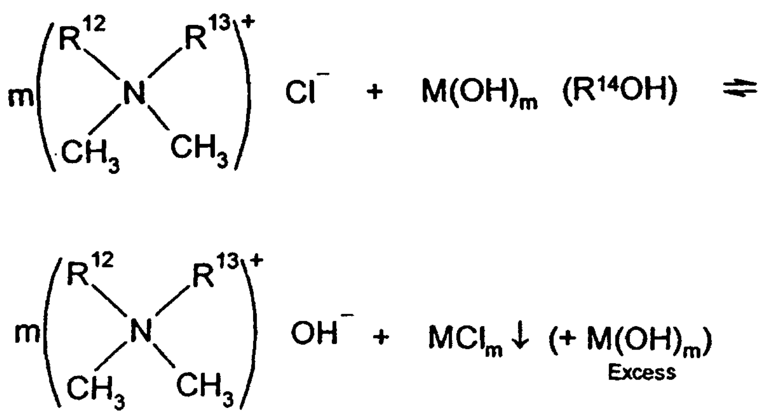

(XXV)

wherein $R^{12}$ is a $C_1$-$C_{20}$ alkyl or aryl-substituted alkyl group; $R^{13}$ is a $C_8$-$C_{20}$ alkyl group; $R^{14}$ is a straight chain $C_1$-$C_4$ alkyl group; M is a mono-, di-, or trivalent metal; and m is one if M is monovalent, two if M is divalent, and three if M is trivalent. Preferably $R^{12}$ is the same as $R^{13}$, i.e. a $C_8$-$C_{12}$ alkyl group.

Many $C_1$-$C_{20}$ alkyl or aryl-substituted alkyl, $C_8$-$C_{20}$ alkyl quaternary ammonium chlorides are suitable reactants, but di $C_8$-$C_{12}$ alkyl quaternary ammonium chloride is preferred, and didecyldimethylammonium chloride, and particularly, di-n-decyldimethylammonium chloride is most preferred. The selections of the $R^{12}$ and $R^{13}$ substituents of the chloride quat reactant are determinative of the hydroxy quat product.

Special mention is also made of processes wherein $R^{12}$ is a methyl, butyl, $C_8$ alkyl, $C_9$ isoalkyl, $C_{10}$ alkyl, $C_{12}$ alkyl, $C_{14}$ alkyl or benzyl group; and $R^{13}$ is a $C_{10}$ alkyl, $C_{12}$ alkyl, $C_{14}$ alkyl or $C_{16}$ alkyl group.

The metal hydroxide reactant is a mono-, bi-, or trivalent metal hydroxide, preferably a monovalent metal hydroxide, and most preferably an alkali metal hydroxide such as sodium hydroxide or potassium hydroxide. Special mention is made of potassium hydroxide. The metal chloride reaction product will precipitate and is easily removed, i.e. by filtration or the like, yielding a hydroxy quat/solvent reaction product. The hydroxy quat can be separated therefrom by drying or the like.

The reaction is conducted in a solvent which comprises a $C_1$-$C_4$ normal alcohol. Preferably, the solvent is ethanol, and most preferably, anhydrous ethanol.

The amount of metal hydroxide reactant typically is a stoichiometric amount with respect to the quaternary ammonium chloride reactant. Therefore, on a theoretical basis and if the reaction were complete and unequilibrated, there would be no excess of metal hydroxide reactant upon completion of the reaction. In practice, yield when using a stoichiometric amount of metal hydroxide reactant will range from about 65% to about 95%, but will vary, dependent in part upon the particular metal hydroxide reactant.

Yield can be further improved over conventional methods by utilization of a stoichiometric excess of metal hydroxide ranging from about 2% to about 20% excess. If an excess of metal hydroxide is used, yield will be increased to from about 95% to about 99%, again varying as above.

The unreacted metal hydroxide is soluble in the hydroxy quat/solvent mixture. Any excess or unreacted metal hydroxide should be removed after the reaction is completed, and is preferably precipitated by subsequent reaction with carbon dioxide to yield the corresponding metal carbonate. The carbonate is insoluble in the hydroxy quat/solvent mixture and is easily removed, i.e. by filtration or the like. Alternatively, a solid metal bicarbonate, in which the metal corresponds to the metal of the metal hydroxide, can be added and slurried with the hydroxy quat/solvent mixture. The soluble metal hydroxide reacts with solid bicarbonate to yield the insoluble metal carbonate. The metal carbonate does not react further with the hydroxy quat.

Mixing, adding, and reacting of the components in the preparation of these hydroxy quats can be accomplished by conventional means known to those of ordinary skill in the art. The order of addition of reactants or solvent does not affect the process. Reactants and/or solvent can be added sequentially or simultaneously in any suitable reaction vessel.

Typically, the reactants and solvent will be stirred and heated to from about 20°C to about 70°C and held at that temperature for a period of from about 1 hour to about 5 hours. The reaction mixture is then cooled, first to room temperature and then to about 0°C where it is held for about 1 hour to about 2 hours. Any precipitated metal chloride is collected as is known in the art, i.e. such as by filtration.

Alternatively, the reactants and solvent can be stirred at a slightly elevated temperature, i.e. from about 20°C to about 40°C, to yield the hydroxy quat/solvent mixture. Hydroxy quat can be separated as above.

Quaternary Ammonium Carbonate

Although any quaternary ammonium carbonates are suitable for use in the present invention, preferred carbonate quats have the formula

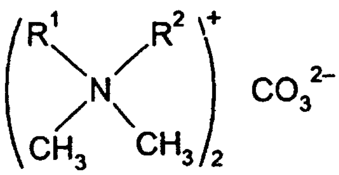

(XI)

wherein $R^1$ is a $C_1$-$C_{20}$ alkyl or aryl-substituted alkyl group, $R^2$ is a $C_8$-$C_{20}$ alkyl group, and preferably $R^1$ and $R^2$ are the same $C_8$-$C_{12}$ alkyl group.

Special mention is made of carbonate quats wherein $R^1$ is a methyl, $C_8$ alkyl, $C_9$ isoalkyl, $C_{10}$ alkyl, $C_{12}$ alkyl, $C_{14}$ alkyl, $C_{16}$ alkyl, or benzyl group; and $R^2$ is a $C_{10}$ alkyl, $C_{12}$ alkyl, $C_{14}$ alkyl, or $C_{16}$ alkyl group.

Most preferred carbonate quats are didecyldimethylammonium carbonate wherein $R^1$ and $R^2$ are a $C_{10}$ alkyl group and preferably an n-$C_{10}$ alkyl group. Didecyldimethylammonium carbonate, when observed as a 70-80 percent by weight solution is a yellow/orange liquid that has a slightly fruity odor. This formulation has a flash point of about 71 °C (160 °F), and it reacts with carboxyl containing compounds.

One or more of these carbonate quats alone or in combination with the corresponding bicarbonate quat(s) and/or metal carbonate salt(s), preferably potassium carbonate salt, can be formulated in the present waterproofer, wood preservative systems.

The stability, and particularly the thermal stability, of carbonate quats is superior to that of hydroxy quats, making these carbonate quats suitable for concentrating and as stock intermediates for further processing. different uses as known to those of ordinary skill in the art, metal stabilizers are not required and, in fact, are not recommended to inhibit leaching of the quat from the substrate. Accordingly, wood substrates, such as lumber, timber, and the like, can be treated with metal coupler-free preservative systems which comprise the above carbonate quat(s) diluted in a suitable solvent as above.

The amount of di $C_8$-$C_{12}$ alkyl quaternary ammonium carbonate(s) used to treat the substrate is a biocidal effective amount, i.e. that amount effective to inhibit the growth of or to kill one or more organism that causes wood rot, to inhibit sap stain, or a combination thereof. Such organisms include, but are not limited to, Trametes viride or Trametes versicolor, which cause a white rot; Goeophyllium trabeum, which causes a brown rot; and Aspergillus niger, which causes sap stain/mold.

Typically, a wood preservative system will comprise from about 0.1 to about 5 parts by weight of the carbonate quat(s) and from about 95 to about 99.9 parts by weight of solvent based upon 100 parts by weight of quat and solvent combined. Most preferably, the wood preservative system of the present invention will comprise from about 1 to about 2 parts by weight of carbonate quat(s) and from about 98 to about 99 parts by weight of solvent on the same basis.

Treatment of the substrate is accomplished by any means known to those of ordinary skill in the art including, but not limited to, dipping, soaking, brushing, pressure treating, or the like. The length of treatment required will vary according to treatment conditions, the selection of which are known to those skilled in the art.

These metal coupler-free preservative systems display greater resistance to leaching than wood preservatives currently used in the industry. Resistance to leaching is defined as retention of a biocidal effective amount, and preferably at least about 2% by weight, of carbonate quat(s) in the substrate over a prolonged period of at least about 100

Although certain carbonate quats can be prepared by a variety of methods, applicants have discovered an indirect synthesis method that can be used to prepare a variety of $C_1$-$C_{20}$ alkyl or aryl-substituted alkyl, $C_8$-$C_{20}$ alkyl quaternary ammonium carbonate compounds, preferably di $C_8$-$C_{12}$ alkyl quaternary ammonium carbonate compounds, and most preferably didecyldimethylammonium carbonate, according to the following scheme:

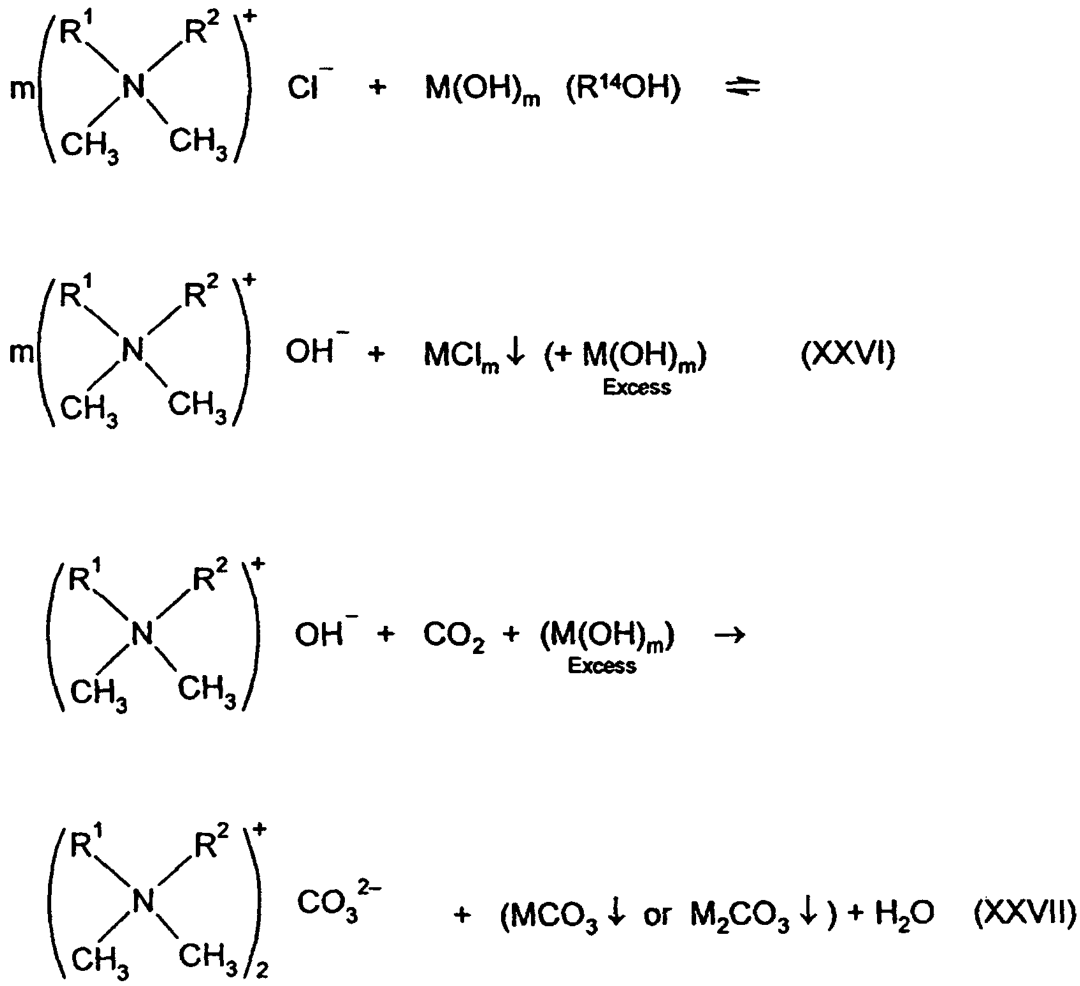


(XXVI)

(XXVII)

wherein $R^1$ is a $C_1$-$C_{20}$ alkyl or aryl-substituted alkyl group; $R^2$ is a $C_8$-$C_{20}$ alkyl group; and preferably $R^1$ is the same as $R^2$ and $R^1$ is a $C_8$-$C_{12}$ alkyl group; $R^{14}$ is a straight chain $C_1$-$C_4$ alkyl group; M is a mono-, bi-, tri-valent metal, preferably a monovalent metal, and most preferably an alkali metal; and m is 1 if M is mono-valent, 2 if M is di-valent, and 3 if M is trivalent.

A $C_1$-$C_{20}$ alkyl or aryl-substituted alkyl, $C_8$-$C_{20}$ alkyl, and preferably a di $C_8$-$C_{12}$ alkyl, quaternary ammonium chloride is used as a starting material and is reacted with a metal hydroxide to yield a $C_1$-$C_{20}$ alkyl or aryl-substituted alkyl, $C_8$-$C_{20}$ alkyl, and preferably a di $C_8$-$C_{12}$ alkyl, quaternary ammonium hydroxide intermediate. The hydroxy quat intermediate(s) and any excess metal hydroxide are then reacted with carbon dioxide to yield the carbonate quat(s) and the metal carbonate.

Many di $C_8$-$C_{12}$ alkyl quaternary ammonium chlorides are suitable reactants to prepare the intermediate hydroxy quat and are described above. The selections of the $R^1$ and $R^2$ substituents of the chloride quat reactant are determinative of the hydroxy quat intermediate, and therefore, of the carbonate quat product.

The metal hydroxide reactant is also as described above.

The metal chloride first step reaction product will precipitate and is easily removed, i.e. by filtration or the like, yielding a hydroxy quat/solvent reaction product. The hydroxy quat can be separated therefrom by drying or the like, if desired.

The first reaction (XXVI) is conducted in a solvent as described above, and the amount of metal hydroxide reactant is as described above.

Hydroxy quat and any unreacted metal hydroxide are then reacted with at least a stoichiometric equivalent of carbon dioxide to yield the quaternary ammonium carbonate(s), and if any unreacted metal hydroxide is present, the metal carbonate(s). The conversion of the metal hydroxide to the metal carbonate is the preferred reaction of the two carbonations and will proceed more rapidly. The metal carbonate will precipitate and can be separated easily, i.e. by filtration or the like, leaving the stable carbonate quat (s) or carbonate quat(s)/solvent reaction product.

The carbonation step can also produce the bicarbonate quat or the metal carbonate quat as byproducts. The carbonate quat alone or in combination with the bicarbonate quat and/or the metal carbonate quat are suitable for use in the metal coupler-free wood preservative systems of the present invention. These carbonate quats or carbonate/bicarbonate/metal carbonate compositions, do not require a metal coupler for stabilization in a wood substrate. Completely metal-free wood preservative systems are preferred. However, if a metal carbonate quat is included in the system, preferably the metal is not a metal currently used as a coupler, and most preferably, it is an alkali metal and does not pose environmental or corrosion hazards or concerns.

Mixing, adding, and reacting of the components in the preparation of these carbonate quats can be accomplished by conventional means known to those of ordinary skill in the art. The order of addition of reactants or solvent in any individual step does not affect the process. Reactants and/or solvent can be added sequentially or simultaneously in any suitable reaction vessel. For example, the metal hydroxide may be dissolved in alcohol and the resultant mixture added to the chloride quat or the chloride quat may be dissolved in alcohol and the metal hydroxide added to the resultant mixture.

The carbon dioxide is generally bubbled for a suitable period known to those of ordinary skill in the art through the hydroxy quat/solvent supernatant after the metal chloride precipitate has been separated. Alternatively, the carbon dioxide can be added as solid dry ice directly to the hydroxy quat. Typically, this time varies from about 0.5 hour to about 1 hour at ambient temperature. Any precipitated metal carbonate is collected as is known in the art, i.e., such as by filtration.

<u>Quaternary Ammonium Carboxylate</u>

Although any quaternary ammonium carboxylates are suitable for use in the present invention, preferred carboxylate quats have the formula

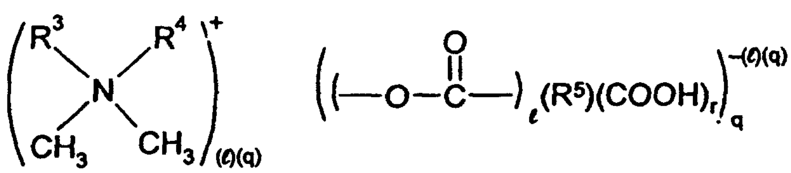

(XXVIII)

wherein $R^3$ is a $C_1$-$C_{20}$ alkyl or aryl-substituted alkyl group; $R^4$ is a $C_8$-$C_{20}$ alkyl group; but preferably $R^3$ and $R^4$ are the same $C_8$-$C_{12}$ alkyl group; $R^5$ is a substituted or unsubstituted, interrupted or uninterrupted $C_1$-$C_{100}$ group; $\ell$ and q independently are 1, 2, or 3, and $(\ell)(q)$ is 1, 2, or 3; and r is 0 or an integer from 1 to 50.

Special mention is also made of carboxylate quats wherein $R^3$ is a methyl, $C_8$ alkyl, $C_9$ isoalkyl, $C_{10}$ alkyl, $C_{12}$ alkyl, $C_{14}$ alkyl or benzyl group; and $R^4$ is a $C_{10}$ alkyl, $C_{12}$ alkyl, $C_{14}$ alkyl or $C_{16}$ alkyl group. Most preferred carboxylate quats are didecyldimethylammonium carboxylates wherein $R^3$ and $R^4$ are a $C_{10}$ alkyl group and most preferably an n-$C_{10}$ alkyl group.

Preferred carboxyl anions are derived from saturated or unsaturated mono- or poly-, including; but not limited to, di- or tri-, carboxylic acids, and particularly $C_1$-$C_{20}$ carboxylic acids, or anhydrides thereof. $R^5$ independently can be substituted, particularly by one or more oxygen or boron atoms or sulfate groups, or interrupted, particularly by one or more oxygen or boron atoms or sulfate groups. Special mention is made of acetic acid, gluconic acid, lauric acid,

formic acid, propionic acid, butyric acid, oxalic acid, acrylic acid, tartaric acid, benzoic acid, octanoic acid, and the like. Additionally, the carboxyl group can be derived from polymeric acids or copolymers in which one or more of the monomers is an acid. An example of a polyacid is polyacrylic acid. Examples of copolymer acids include, but are not limited to, olefin/carboxylic acid polymers such as poly(ethylene/acrylic acid).

Such acids, including the polymeric or copolymeric acids mentioned above are of the formula

$$((R^5(COOH)_{(\ell+r)})_q \qquad (XXIX)$$

where $R^5$, $\ell$, r, and q are defined as above. In polymeric copolymers carboxylic acids, $R^5$ can be represented as $((R^{15})_s(R^{16})_t)$ giving

$$((R^{15})_s(R^{16})_t(COOH)_{(\ell+r)})_q \qquad (XXX)$$

where $R^{15}$ and $R^{16}$ independently are substituted or unsubstituted, interrupted or uninterrupted as above $C_1$-$C_{100}$ groups and a and t independently are integers from 1 to 100. Preferably, $R^5$, $R^{15}$, and $R^{16}$ independently are alkyl or alkenyl groups.

Although the carboxylate quats can be prepared by a variety of methods, preferably they are prepared by an indirect synthesis, a direct synthesis, or a hydroxy quat/acid synthesis.

The indirect synthesis is illustrated below

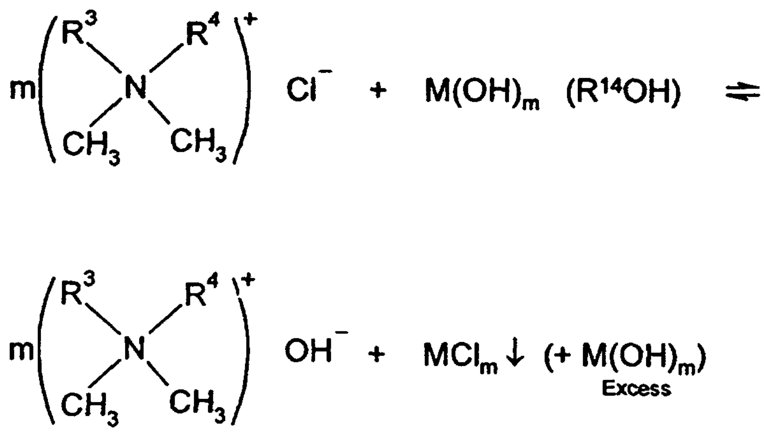


(XXXI)

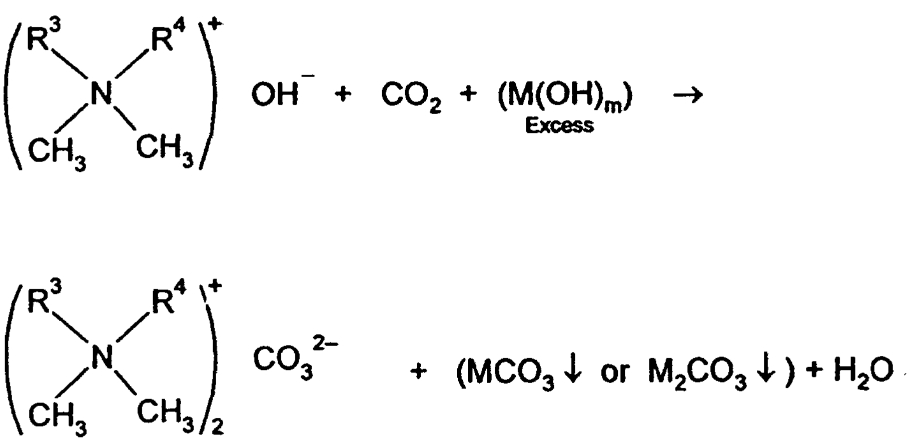


(XXXII)

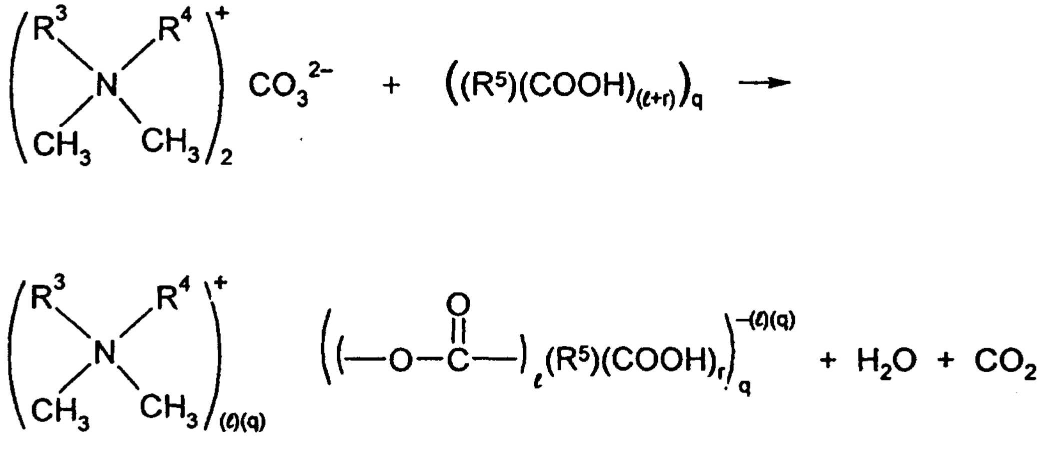

(XXXIII)

wherein $R^3$ is a $C_1$-$C_{20}$ alkyl or aryl-substituted alkyl group; $R^4$ is a $C_8$-$C_{20}$ alkyl group; and preferably $R^3$ is the same as $R^4$ and $R^4$ is a $C_8$-$C_{12}$ alkyl group; $R^{14}$ is a straight chain $C_1$-$C_4$ alkyl group; $R^5$ is a substituted or unsubstituted, as explained above, interrupted or uninterrupted, as explained above, $C_1$-$C_{100}$ group; $\ell$ and q independently are 1, 2, or 3 and (t)(q) is 1, 2, or 3; M is a mono-, bi-, tri-valent metal, preferably a monovalent metal, and most preferably an alkali metal; r is 0 or an integer from 1 to 50; and m is 1 if M is mono-valent, 2 if M is di-valent, and 3 if M is tri-valent.

The carboxylate quat is prepared via a carbonate quat intermediate.

A $C_1$-$C_{20}$ alkyl or aryl-substituted alkyl, $C_8$-$C_{20}$ alkyl, and preferably a di $C_8$-$C_{12}$ alkyl, quaternary ammonium chloride is used as a starting material and is reacted with a metal hydroxide to yield a $C_1$-$C_{20}$ alkyl or aryl-substituted alkyl, $C_8$-$C_{20}$ alkyl, and preferably a di $C_8$-$C_{12}$ alkyl, quaternary ammonium hydroxide intermediate as above. The hydroxy quat intermediate(s) and any excess metal hydroxide are then reacted with carbon dioxide to yield the carbonate quat(s) and the metal carbonate(s) as above. The carbonate quat second intermediate(s) is then reacted with at least one carboxylic acid to yield the carboxylate quat(s). The selection of the $C_8$-$C_{12}$ alkyl substituent of the chloride quat reactant is determinative of the hydroxy quat first intermediate, therefore, of the carbonate quat second intermediate, and ultimately, of the cation component of the carboxylate quat product.

The metal hydroxide reactant is described above. The preparation of the hydroxy quat is preferably conducted in a solvent as described above, and the amount of metal hydroxide reactant is described above.

Hydroxy quat and any unreacted metal hydroxide are then reacted with carbon dioxide to yield the quaternary ammonium carbonate(s) as detailed above. The carbonation step can also produce the bicarbonate quat(s) or the metal carbonate quat(s) as by-products.

The carbonate quat second intermediate(s) is then reacted with at least a stoichiometric amount of carboxylic acid(s) to yield the carboxylate quat(s).

The carboxylic acid(s) in reaction (XXXIII) is typically added over a short period of several minutes, and the reaction typically is rapid. The carboxylate quat(s) can be separated or concentrated by filtration or evaporation after a carbon dioxide evolution in this step is completed.

In the indirect synthesis, any acid having a pKa less than that of carbonic acid, i.e., less than 6.4, such as carboxylic, phosphoric, sulfonic acids, and the like, can be reacted with a carbonate quat and displace carbon dioxide.

The addition of ammonia will retard the carbonate quat and acid reaction (XXXIII). For example, if ammonia is added to a mixture of a polyacid and a carbonate quat, the acid-carbonate quat reaction is retarded. However, when ammonia is slowly evaporated, the reaction liberating carbon dioxide may proceed, yielding a compound that is fixed (insoluble) in wood. Similarly, a system of polyacid and acetic acid should yield an insoluble polyacid quat when the acetic acid evaporates.

Alternatively, the carboxylate quats can be prepared by a direct synthesis method. A metal salt of a carboxylic acid is reacted with a $C_1$-$C_{20}$ alkyl or aryl-substituted alkyl, $C_8$-$C_{20}$ alkyl, and preferably a di-$C_8$-$C_{12}$ alkyl, quaternary ammonium chloride, in a double replacement reaction, to yield the carboxylate quat and the metal chloride salt

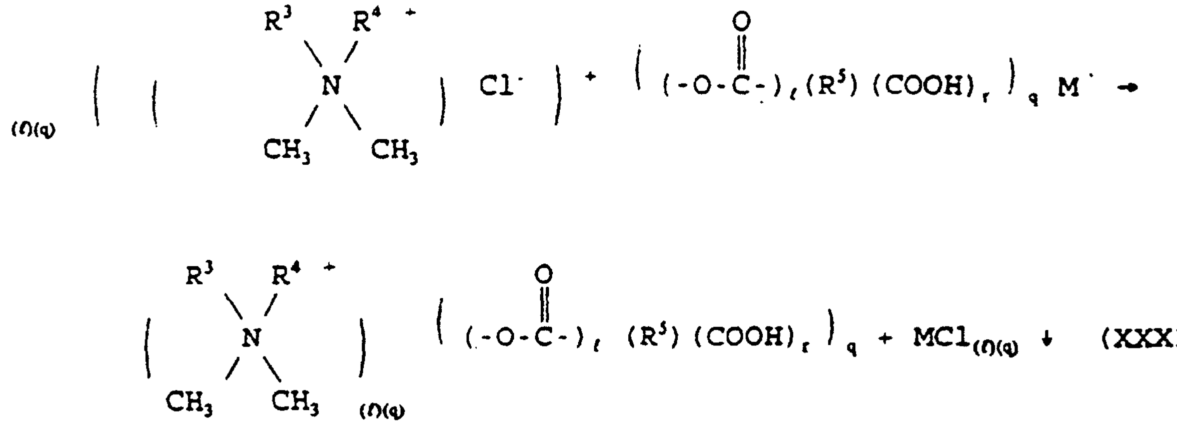

(XXXIV)

wherein $R^3$, $R^4$, $R^5$, M, $\ell$, q, and r are as defined above.

The metal carboxylates are derived from carboxylic acids. The carboxylic acids are as described and detailed above. The metals are mono-, di-, or the tri-valent metals, preferably mono-valent metals and most preferably alkali metals. Special mention is made of potassium and sodium.

Reaction (XXXIV) can be conducted neat or in a number of solvents including, but not limited to ethanol, acetic acid, or propionic acid. Preferably, the solvent comprises a $C_1$-$C_4$ normal alcohol as described above. Yield will depend on the solvent and the reaction conditions selected, which can be determined by one of ordinary skill in the art through routine experimentation in accordance with this detailed explanation.

The chloride quat starting material is selected as above and again, its selection is determinative of the cation of the carboxylate quat to be formed.

Finally, a third method for the production of the carboxylate quat(s) includes reacting hydroxy quat(s) with carboxylic acid(s).

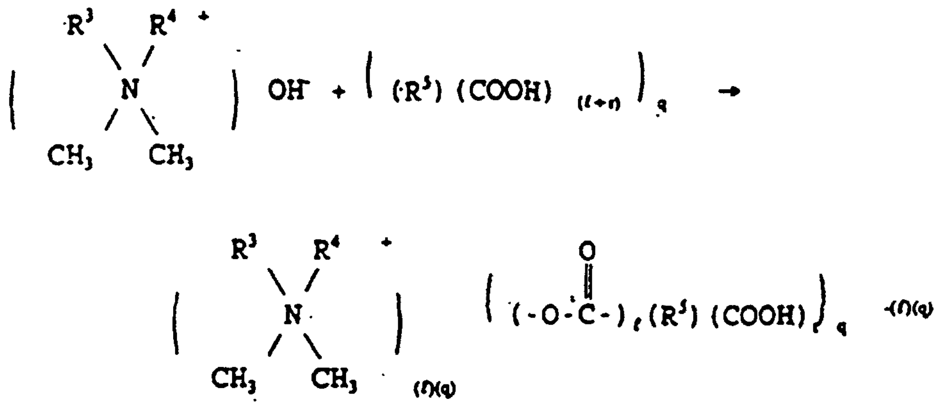

(XXXV)

wherein $R^3$, $R^4$, $R^5$, $\ell$, 1, and r are as defined above.

The hydroxy quat(s), carboxylic acid(s), and carboxylate quat(s) are as described above.

Mixing, adding, and reacting of the components in any of the direct, indirect or hydroxy quat/acid methods can be accomplished by conventional means known to those of ordinary skill in the art. The order of addition of reactants or solvent in any individual step does not affect the process.

Quaternary Ammonium Borate

Although any quaternary ammonium borates are suitable for use in the present invention, preferred borate quats have the formula

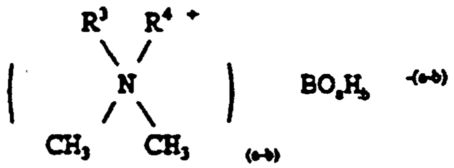 (XXXVI)

wherein $R^3$ is a $C_1$-$C_{20}$ alkyl or aryl-substituted alkyl group; $R^4$ is a $C_8$-$C_{20}$ alkyl group; but preferably $R^3$ and $R^4$ are the same $C_8$-$C_{12}$ alkyl group; a is 2 or 3, but when a is 2, b is 0 or 1 and when a is 3, b is 0, 1, or 2..

Special mention is also made of borate quats wherein $R^3$ is a methyl, $C_8$ alkyl, $C_9$ isoalkyl, $C_{10}$ alkyl, $C_{12}$ alkyl, $C_{14}$ alkyl or benzyl group; and $R^4$ is a $C_{10}$ alkyl, $C_{12}$ alkyl, $C_{14}$ alkyl or $C_{16}$ alkyl group. Most preferred borate quats are didecyldimethylammonium borates wherein $R^3$ and $R^4$ are a $C_{10}$ alkyl group and most preferably an n-$C_{10}$ alkyl group.

Typically, the production of the borate quat(s) includes reacting hydroxy quat(s) with boric acid.

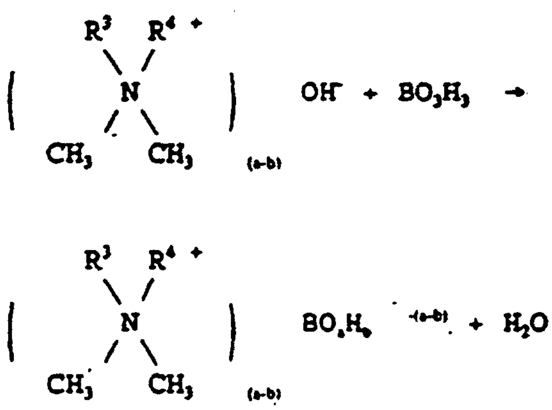 (XXXVII)

wherein $R^3$, $R^4$, a, and b are as defined above.

Mixing, adding, and reacting of the components in the hydroxy quat/acid method can be accomplished by conventional means known to those of ordinary skill in the art. The order of addition of reactants does not affect the process.

<u>Waterproofers</u>

The polyhydroxyl or polyether hydroxyl fatty acid ester or the polyether hydroxide waterproofers of the present invention are soluble in both aqueous and organic solvent systems. Furthermore, they render the water-soluble quats described herein useful in aqueous or organic systems as well. This occurs despite the fact that these quats alone, i.e. without the present waterproofers, are relatively insoluble in organic solvents, emulsions or dispersions., i.e. they are not generally useful in preserving wood when in an organic solvent.

The waterproofers of the present invention include compositions of the formula:

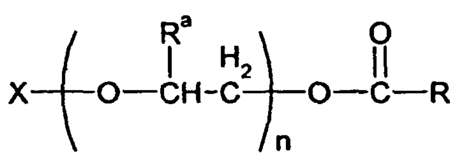 (XXXVIII)

wherein:

X is hydrogen or

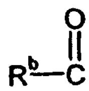 ;

R and $R^b$ independently are a saturated or unsaturated $C_9$-$C_{50}$ group;
$R^a$ is hydrogen or a methyl group; and
n is an integer from 1 to 10.

Saturated $C_9$-$C_{50}$ groups include $C_9$-$C_{50}$ straight chain, branched, or cycloalkyl groups. Unsaturated $C_9$-$C_{50}$ groups include those groups having one or more double or triple bonds or combinations thereof including acyclic groups (including straight chain or branched), cyclic groups, or combinations thereof. In combinations, unsaturation may occur in the cyclic portion, the acyclic portion, or both.

Special mention is made of

(A) propylene glycol monostearate, wherein X is hydrogen, R is a $C_{17}$ alkyl group, $R^a$ is a methyl group and n is 1;
(B) polyethylene glycol distearate (PEG 400-DS) wherein X is

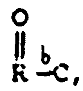

R and $R^b$ each are a $C_{17}$ alkyl group, $R^a$ is hydrogen, and n is 8; and
(C) glycol monostearate wherein X is hydrogen, R is a $C_{17}$ alkyl group, $R^a$ is hydrogen, and n is 1.

The fatty acid ester compounds used as waterproofer are known as stabilizers in disinfectant waxemulsions (DE 29 16 304 A1) or as possible nonionic surfactants in tannin extract based wood preservatives (US 4,732,817) or rosin based wood preservatives (WO 87/06177 A1).

Also contemplated by the present invention are combinations of any of the above waterproofers.

These waterproofers hinder migration of the quat molecules from a substrate under wet conditions. Furthermore, where surface corrosion problems are related to the water holding properties of the quat, the waterproofer displaces or prevents the entry of water.

Solvents

The waterproofer and waterproofer, preservative systems of the present invention may include a suitable solvent including aqueous and non-aqueous solvents. Preferably, the solvent is an aqueous solvent including, but not limited to, water, aqueous alcohol, ammonia water, aqueous acetic acid, and the like, or a combination of any of the foregoing. Organic solvents may also be used. These include, but are not limited to, mineral spirits-based solvents and the like.

Waterproofer Systems and Treatment of Substrates

The amount of waterproofer used in the waterproofer systems of the present invention is a waterproofing enhancing amount, i.e. that amount effective to impart or to increase the water resistance of a substrate treated therewith.

Typically, a waterproofer system will comprise from about 0.1 to about 20 parts by weight of waterproofer and from about 80 to about 99.9 parts by weight of solvent based upon 100 parts by weight of waterproofer and solvent combined. Preferably, the waterproofer system of the present invention will comprise from about 0.2 to about 5 parts by weight of waterproofer and from about 95 to about 99.8 parts by weight of solvent on the same basis.

The components of the waterproofer systems of the present invention are mixed by conventional means known to those skilled in the art. Other conventional additives may be added as required for application to different substrates and for different uses as known to those of ordinary skill in the art. Wood substrates, such as lumber, timber, or the like, can be treated with these systems. Treatment of the substrate is accomplished by any means known to those of ordinary skill in the art including, but not limited to, dipping, soaking, brushing, pressure treating, or the like. The length of treatment time required will vary according to treatment conditions, the selection of which are known to those skilled in the art.

Waterproofer, Wood Preservative Systems and Treatment of Substrates

The amount of waterproofer used in the waterproofer, wood preservative systems of the present invention is a waterproofing and compatibilizing enhancing amount, i.e. that amount effective to impart or to increase the water resistance, leaching resistance, and/or dimensional stability of the waterproofer, wood preservative system and/or the quat and to enhance the compatibility of the quats of the present invention with a solvent.

The amount of quaternary ammonium composition(s) is a biocidal effective amount, i.e. that amount effective to inhibit the growth of or to kill one or more organism that causes wood rot, to inhibit sap stain, or a combination thereof. Such organisms include, but are not limited to, Trametes viride or Trametes versicolor, which cause a white rot; Gloeophyllum trabeum, which causes a brown rot; and Aspergillus niger, which causes sap stain/mold.

Typically, a waterproofer, wood preservative system will comprise from about 0.1 to about 15 parts by weight of waterproofer(s), from about 0.1 to about 10 parts by weight of quat(s), and from about 99.8 to about 75 parts by weight of solvent based upon 100 parts by weight of quat, waterproofer, and solvent combined. Preferably, the waterproofer, wood preservative systems of the present invention will comprise from about 0.5 to about 6 parts by weight of quat(s) from about 0.5 to about 8.5 parts by weight of waterproofer(s), and from about 96 to about 85.5 parts by weight of solvent on the same basis.

The components of the waterproofer, wood preservative systems of the present invention are mixed by conventional means known to those skilled in the art preferably to form an emulsion. Preferably, the waterproofer and the quat are melted together. The melt can then be stirred, and warm water (about 40 to 50°C) added with stirring to yield an emulsion or solution. Emulsions prepared in this manner may be stable for periods of at least one year.

Although other conventional additives including, but not limited to, emulsifiers may be added as required for application to different substrates and for different uses as known to those of ordinary skill in the art, metal stabilizers are not required and, in fact, are not recommended to inhibit leaching of the quat from the substrate. Accordingly, wood substrates, such as lumber, timber, or the like, can be treated with these systems.

Treatment of the substrate is accomplished by any means known to those of ordinary skill in the art including, but not limited to, dipping, soaking, brushing, pressure treating, or the like. The length of treatment required will vary according to treatment conditions, the selection of which are known to those skilled in the art.

The waterproofer, wood preservative systems of the present invention display greater resistance to leaching and greater waterproofing properties, as indicated by swell index, than wood preservatives currently used in the industry. Resistance to leaching is defined as retention of a biocidal effective amount, and preferably at least about 2% by weight, of quat in the substrate over a prolonged period of at least about 100 hours and preferably about 350 hours. Although any positive swell index indicates some waterproofing ability, a swell index of greater than about 50 indicates notable water proofing properties .

DESCRIPTION OF THE PREPERRED EMBODIMENTS

The following examples 1 - 38 illustrate preparation of compounds. Examples 39-69 illustrate use of waterproofers in accordance with this invention. All parts and percentages are given by weight unless otherwise indicated.

Quaternary ammonium compounds are quantified by two phase titration with sodium laurylsulfate and an indicator. The mixture is buffered to a pH of 10.

Swell index is calculated as

$$\left( \frac{\text{(Swell of Control - Swell of Sample)}}{\text{Swell of Control}} \right) \times 100$$

PREPARATION OF HYDROXY OUATS

Example 1 - Stoichiometric Amount of Metal Hydroxide

180 g (0.4 mol) of 80% didecyldimethylammonium chloride in 20% ethanol water (144 g of DDAC), 180 ml of absolute denatured ethanol (denatured with methanol/isopropanol), and 26 g (0.4 mol) of 85% potassium hydroxide pellets (22.1 g of KOH) were mixed in a flask that was purged with nitrogen and equipped with a heating mantle and a magnetic stirrer. The mixture was stirred and heated at 60-70°C for three hours. The mixture was then allowed to cool to room temperature and finally cooled to 0°C for at least one hour.

Potassium chloride precipitated, and the precipitate was collected on a vacuum filter. The solid was washed

with cold ethanol and subsequently was dried, yielding 30 g of dry potassium chloride. The quat solution was concentrated in a vacuum to about 75% active bases.

Yield was 180 g of product containing 138 grams of didecyldimethylammonium hydroxide.

Example 2

The procedure of Example 1 was followed, but the mixture was stirred mechanically at 50°C for one hour. Potassium chloride precipitated, and the precipitate was collected on a vacuum filter. The solid was washed with cold ethanol and subsequently was dried, yielding 30 g of dry potassium chloride.

Yield was 180 g of product containing 138 g of didecyldimethylammonium hydroxide.

Example 3

0.022 mol of 85% potassium hydroxide pellets (1.23 g of KOH) was added to 0.022 mol of 80% didecyldimethylammonium chloride in 20% ethanol/water ( 8 g of DDAC) dissolved in 10 ml of ethanol. The resultant mixture was stirred and heated to 70°C and held at this temperature for one-half hour. The pellets dissolved, and a fine precipitate formed. The mixture was then cooled and chilled to 0°C. The precipitated solid was collected on a filter and washed with cold ethanol. The filtrate was concentrated to yield a yellow/orange oil with a slight amine odor.

Results are summarized in Table 1.

Example 4

0.022 mol of 85% potassium hydroxide pellets (1.23 g of KOH) was added to 0.022 mol of 80% didecyldimethylammonium chloride in 20% ethanol/water (8 g of DDAC) dissolved in 10 ml of propanol. The resultant mixture was stirred and heated to 80°C and held at this temperature for one hour. The pellets dissolved, and a fine precipitate formed. The mixture was then cooled and chilled to 0°C. The precipitated solid was collected on a filter and washed with cold ethanol. The filtrate was concentrated to yield a yellow/orange oil with a slight amine odor.

Results are illustrated in Table 1.

Example 5

The procedure of Example 3 was followed substituting sodium hydroxide for the potassium hydroxide.

Results are illustrated in Table 1.

Example 6

The procedure of Example 4 was followed substituting sodium hydroxide for the potassium hydroxide.

Results are illustrated in Table 1.

TABLE 1

| Preparation of Didecyldimethylammonium Hydroxide from Stoichiometric Amounts of Reactants | | | | |
|---|---|---|---|---|
| Example | 3 | 4 | 5 | 6 |
| Hydroxide | KOH | KOH | NaOH | NaOH |
| Solvent | Ethanol | n-Propanol | Ethanol | n-Propanol |
| Conversion % | 96 | 95 | 81 | 83 |

Examples 3-6 demonstrate that the use of a normal $C_1$-$C_4$ alcohol as a reaction medium enhances conversion of the chloride quat to the hydroxy quat. Furthermore, a comparison of examples 3 and 4 with Examples 5 and 6 illustrates the increase in conversion by the use of the preferred metal hydroxide, potassium hydroxide.

Stoichiometric Excess of Metal Hydroxide

Example 7

A nitrogen purged reactor equipped with a heating mantle and a magnetic stir bar was charged with 0.4 mol

of 80% didecyldimethylammonium chloride (144 g of DDAC) in 20% ethanol/water, 180 ml of ethanol, and 0.49 mol of 85% potassium hydroxide ( 27.5 g of KOH) pellets. The mixture was heated at 60-70°C for 3 hours, allowed to cool to room temperature, and then cooled to 0°C for about one hour to precipitate potassium chloride. The precipitate was collected on a vacuum filter, and the solid was washed with cold ethanol. Potassium chloride yield was 30.8 g.

The supernatant solution, which contained the hydroxy quat and 0.09 mol of excess potassium hydroxide, was stirred with 2 g (0.045 mol) of carbon dioxide gas (from dry ice). The mixture was kept cold for an hour and then was vacuum filtered to remove 7.2 g (theoretical 6.2 g) of potassium carbonate.

Conversion percentage to the hydroxy quat was determined to be 99%.

Preparation of Carbonate Quats

Example 8

180 g (0.4 mol) of 80% didecyldimethylammonium chloride in 20% ethanol water (144 g DDAC), 180 ml of absolute denatured ethanol (denatured with methanol/isopropanol), and 32 g (0.49 mol) of 85% potassium hydroxide pellets (27 g KOH) were mixed in a flask that was purged with nitrogen and equipped with a heating mantle and a magnetic stirrer. The mixture was stirred and heated at 60-70°C for three hours. The mixture was then allowed to cool to room temperature and finally cooled to 5°C.

Potassium chloride precipitated, and the precipitate was collected on a vacuum filter. The solid was washed with cold ethanol and subsequently was dried, yielding 31 g (calculated yield 29.6 g) of dry potassium chloride.

The ethanolic solution of the hydroxy quat containing about 0.09 mol of unreacted KOH, was stirred while 50 g of carbon dioxide (from sublimed carbon dioxide) were bubbled over one half hour. The resultant mixture was then filtered to remove 7.2 g of potassium carbonate (6.2 g calculated), and the filtrate was concentrated to yield an orange/brown liquid with 80-85% carbonate quat in water/ethanol and less than 0.1% chloride quat having a product with 98 to 99% exchanged quat purity.

Example 9

180 g (0.4 mol) of 80% didecyldimethylammonium chloride in 20% ethanol water (144 g DDAC), 180 ml of absolute denatured ethanol (denatured with methanol/isopropanol), and 32 g (0.49 mol) of 85% potassium hydroxide pellets (27 g KOH) were mixed in a flask that was purged with nitrogen and equipped with a heating mantle and a magnetic stirrer. The mixture was heated to 50°C and stirred for one hour.

Potassium chloride precipitated, and the precipitate was collected on a vacuum filter. The solid was washed with cold ethanol and subsequently was dried, yielding 31 g (calculated yield 29.6 g) of dry potassium chloride.

The ethanolic solution of the hydroxy quat containing about 0.09 mol of unreacted KOH, was stirred while 50 g of carbon dioxide (from sublimed carbon dioxide) were bubbled over one half hour. The resultant mixture was then filtered, and the filtrate was concentrated to yield an orange/brown liquid. Yield was similar to that of Example 8.

Indirect Synthesis of Carboxylate Quat

Example 10 - Didecyldimethylammonium propionate

180 g (0.4 mol) of 80% didecyldimethylammonium chloride in 20% ethanol water (144 g of DDAC), 180 ml of absolute denatured ethanol (denatured with methanol/isopropanol), and 32 g (0.49 mol) of 85% potassium hydroxide pellets (27 g of KOH) were mixed in a flask that was purged with nitrogen and equipped with a heating mantle and a magnetic stirrer. The mixture was stirred and heated at 60-70°C for three hours. The mixture was then allowed to cool to room temperature and finally cooled to 5°C.

Potassium chloride precipitated, and the precipitate was collected on a vacuum filter. The solid was washed with cold ethanol and subsequently was dried, yielding 31 g (calculated yield 29.6 g) of dry potassium chloride.

The ethanolic solution of the hydroxy quat containing about 0.09 mol of unreacted KOH, was stirred while 50 g of carbon dioxide (from sublimed carbon dioxide) were bubbled over one half hour. The resultant mixture was then filtered to remove 7.2 g of potassium carbonate (6.2 g calculated), and the filtrate was concentrated to yield an orange/brown liquid with 80-85% carbonate quat (0.4 mol of carbonate quat) and less than 0.1% chloride for a product with 98 to 99% exchanged quat purity.

The cold product, after filtration, was placed in a closed flask equipped with a condenser, addition funnel, and a tube connected to a water displacement type gas measuring device. An equivalent (0.4 mol, 29.6 g), of propionic acid was added to the carbonate quat over five minutes. Immediate gas evolution was noted, and 5.75 liters of gas were collected over 15 minutes. The solvent was removed on a rotary evaporator after the carbon dioxide evolution

ceased, and yielded a yellow/orange liquid.

Quat analysis revealed that the product contained 85% active quat with 0.09% free chloride and 99% exchange.

Example 11 - Didecyldimethylammonium acetate

The procedure of Example 10 is followed, substituting 0.4 mol of acetic acid for the propionic acid.

Example 12 - Didecyldimethylammonium 2-ethylhexanoate

The procedure of Example 10 is followed, substituting 0.4 mol of 2-ethylhexanoic acid for the propionic acid.

The product is cloudy.

Example 13 - Didecyldimethylammonium gluconate

The procedure of Example 10 is followed, substituting 0.4 mol of gluconic acid for the propionic acid.

The product is water soluble.

Example 14 - Didecyldimethylammonium octanoate

The procedure of Example 10 is followed, substituting 0.4 mol of octanoic acid for the propionic acid.

Example 15 - Didecyldimethylammonium mixed coconut fatty acid carboxylate

The procedure of Example 10 is followed, substituting 0.4 mol of mixed coconut fatty acid for the propionic acid.

Example 16 - Didecyldimethylammonium laurate

The procedure of Example 10 is followed, substituting 0.4 mol of lauric acid for the propionic acid.

The product is a waxy solid.

Example 17 - Octyldecyldimethylammonium propionate

The procedure of Example 10 was followed, substituting 0.4 mol of 80% octyldecyldimethylammonium chloride for the didecyldimethylammonium chloride to yield octyldecyldimethylammonium propionate.

Example 18 - Octyldecyldimethylammonium acetate

The procedure of Example 11 was followed, substituting 0.4 mol of 80% octyldecyldimethylammonium chloride for the didecyldimethylammonium chloride to yield octyldecyldimethylammonium acetate.

Example 19 - Isononyldecyldimethylammonium 2-ethylhexanoate

The procedure of Example 12 was followed, substituting 0.4 mol of 80% isononyldecyldimethylammonium chloride for the didecyldimethylammonium chloride to yield isononyldecyldimethylammonium 2-ethyl-hexanoate.

Example 20 - Isononyldecyldimethylammonium gluconate

The procedure of Example 13 was followed, substituting 0.4 mol of 80% isononyldecyldimethylammonium chloride for the didecyldimethylammonium chloride to yield isononyldecyldimethylammonium gluconate.

Example 21 - Benzyldodecyldimethylammonium gluconate

The procedure of Example 13 was followed, substituting 0.4 mol of 80% benzyldodecyldimethylammonium chloride for the didecyldimethylammonium chloride to yield benzyldodecyldimethylammonium gluconate.

Example 22 - Benzyldodecyldimethylammonium octanoate

The procedure of Example 14 was followed, substituting 0.4 mol of 80% benzyldodecyldimethylammonium chloride for the didecyldimethylammonium chloride to yield benzyldodecyldimethylammonium octanoate.

Example 23 - A mixture of benzyldodecyl-, benzyltetradecyl-, and benzylhexadecyldimethylammonium mixed coconut fatty acid carboxylate

The procedure of Example 15 was followed, substituting 0.4 mol of 80% of a mixture of benzyldodecyl-, benzyltetradecyl-, and benzylhexadecyldimethylammonium mixed fatty acid benzyldodecyldimethylammonium chloride for the didecyldimethylammonium chloride to yield a mixture of benzyldodecyl-, benzyltetradecyl-, and benzylhexadecyldimethylammonium mixed coconut fatty acid carboxylate.

Example 24 - A mixture of benzyldodecyl-, benzyltetradecyl-, and benzylhexadecyldimethylammonium laurate

The procedure of Example 16 was followed, substituting 0.4 mol of 80% of a mixture of benzyldodecyl-, benzyltetradecyl-, and benzylhexadecyldimethylammonium chloride for the didecyldimethylammonium chloride to yield a mixture of benzyldodecyl-, benzyltetradecyl-, and benzylhexadecyldimethylammonium laurate.

Direct Synthesis of Carboxylate Ouat

Example 25 - Didecyldimethylammonium acetate

180 g (0.4 mol) of 80% didecyldimethylammonium chloride in 20% ethanol water (144 g of DDAC), 180 ml of anhydrous ethanol, and a stoichiometric excess, 47 g (0.48 mol), of anhydrous potassium acetate was mixed in a flask that was purged with nitrogen and equipped with a heating mantle, a magnetic stirrer, and a condenser. The mixture was stirred and heated at 60-70°C for two hours. The insoluble potassium acetate crystals slowly dissolved and a finer solid (KCl) separated. The mixture was then cooled to 0°C and vacuum filtered. The solid washed with cold ethanol to remove 30.7 g of potassium chloride (theoretical 29.6 g). The solution was concentrated, cooled, and filtered to remove 6.5 g of potassium acetate (theoretical 29.6 g).

Additional fine crystals of potassium acetate settled out on standing. By assay, the light yellow liquid product was determined to be 80% quat with 100% exchange.

Example 26 - Didecyldimethylammonium gluconate

0.0221 mol of sodium gluconate and 0.0221 mol of 80% didecyldimethylammonium chloride in water were mixed in a flask. The mixture was heated and held until evolution of carbon dioxide gas ceased.

The resultant quat was analyzed, and conversion was determined to be less than 20%.

Example 27 - Didecyldimethylammonium 2-ethylhexanoate

0.0221 mol of sodium 2-ethylhexanoate and 0.0221 mol of 80% didecyldimethylammonium chloride in water were mixed in a flask. The mixture was heated and held until evolution of carbon dioxide gas ceased.

The resultant quat was analyzed, and conversion was determined to be 77%.

Example 28 - Didecyldimethylammonium laurate

0.4 mol of sodium laurate and 0.4 mol of 80% didecyldimethylammonium chloride in water were mixed in a flask. The mixture was heated to 60°C and held for 1 hour.

The resultant quat was analyzed, and conversion was determined to be 90%

Example 29 - Didecyldimethylammonium propionate

0.0221 mol of sodium propionate and 0.0221 mol of 80% didecyldimethylammonium chloride in 8 g of propionic acid were mixed in a flask. The mixture was heated to 60°C - 80°C and held for 2 hours.

The resultant quat was analyzed, and conversion was determined to be 90%

Example 30 - Didecyldimethylammonium propionate

0.4 mol of potassium propionate and 0.4 mol of 80% didecyldimethylammonium chloride in solid form were mixed in a flask. The mixture was heated to 60°C - 80°C and held for 2 hours.
The resultant quat was analyzed, and conversion was determined to be 91%

Hydroxy Quat/Acid Synthesis

Example 31 - Didecyldimethylammonium propionate

180 g (0.4 mol) of 80% didecyldimethylammonium chloride in 20% ethanol water (144 g of DDAC), 180 ml of absolute denatured ethanol (denatured with methanol/isopropanol), and 26 g (0.4 mol) of 85% potassium hydroxide pellets ( 22 g of KOH) were mixed in a flask that was purged with nitrogen and equipped with a heating mantle and a magnetic stirrer. The mixture was stirred and heated at 60-70°C for three hours. The mixture was then allowed to cool to room temperature and finally cooled to 0°C for at least one hour.
Potassium chloride precipitated, and the precipitate was collected on a vacuum filter. The solid was washed with cold ethanol and subsequently was dried, yielding 30 g of dry potassium chloride.
The hydroxy quat/ethanol solution was mixed with a stoichiometric amount of propionic acid to yield a yellow/orange liquid having a flash point of 41.1 °C (106 °F).

Example 32 - Didecyldimethylammonium borate

The procedure of Example 29 is followed substituting 0.4 mol of boric acid for the propionic acid.
The product is a liquid.

Example 33 - Octyldecyldimethylammonium borate

The procedure of Example 32 was followed, substituting 0.4 mol of 80% octyldecyldimethylammonium chloride for the didecyldimethylammonium chloride to yield octyldecyldimethylammonium borate.

Example 34 - Isononyldecyldimethylammonium borate

The procedure of Example 32 was followed, substituting 0.4 mol of 80% isononyldecyldimethylammonium chloride for the didecyldimethylammonium chloride to yield isononyldecyldimethylammonium borate.

Example 35 - Benzyldodecyldimethylammonium borate

The procedure of Example 32 was followed, substituting 0.4 mol of 80% benzyldodecyldimethylammonium chloride for the didecyldimethylammonium chloride to yield benzyldodecyldimethylammonium borate.

Example 36 - A mixture of benzyldodecyl-, benzyltetradecyl-, and benzylhexadecyldimethylammonium borate

The procedure of Example 32 was followed, substituting 0.4 mol of 80% of a mixture of benzyldodecyl-, benzyltetradecyl-, and benzylhexadecyldimethylammonium chloride for the didecyldimethylammonium chloride to yield a mixture of benzyldodecyl-, benzyltetradecyl-, and benzylhexadecyldimethylammonium borate.

Example 37 - Dihexadecyldimethylammonium borate

The procedure of Example 32 was followed, substituting 0.4 mol of 80% dihexadecyldimethylammonium chloride for the didecyldimethylammonium chloride to yield dihexadecyldimethylammonium borate.

Example 38 - Dodecyltrimethylammonium borate

The procedure of Example 32 was followed, substituting 0.4 mol of 80% dodecyltrimethylammonium chloride for the didecyldimethylammonium chloride to yield dodecyltrimethylammonium borate.

Example 39 - Didecyldimethylammonium chloride (DDAC)/polypropylene glycol monostearate (PGMS)/water

3 parts of didecyldimethylammonium chloride and 2.5 parts of PGMS are melted together and stirred while 94.5 parts of warm (40°C) water are added to yield a stable emulsion which is suitable for waterproofing and preserving wood.

Example 40 - DDAC/3% PGMS/Mineral Spirits

The method of Example 30 is followed substituting 3 parts of PGMS for the PGMS and 84 parts of mineral spirits for the water.

Example 41 - DDAC/6% PGMS/Mineral Spirits

The method of Example 40 is followed substituting 6 parts of PGMS for the PGMS and 91 parts of mineral spirits for the mineral spirits.

Example 42 - DDAC/8% PGMS/Mineral Spirits

The method of Example 40 is followed substituting 8 parts of PMGS for the PGMS and 89 parts of mineral spirits for the mineral spirits.

Example 43 - DDAC/12% PGMS/Mineral Spirits

The method of Example 40 is followed substituting 12 parts of PGMS for the PGMS and 85 pares of mineral spirits for the mineral spirits.

Example 44 - DDAC/9% ethylene glycol monostearate (EGMS)/Water

The method of Example 39 is followed substituting 9 parts of EGMS for the PGMS and 88 parts of water for the water.

Example 45 - DDAC/10% ethylene glycol distearate (EGDS)/Water

The method of Example 44 is followed substituting 10 parts of EGDS for the EGMS and 87 parts of water for the water.

Example 46 - DDAC/9% polyethylene glycol distearate (PEG 400-DS)/Water

The method of Example 39 is followed substituting 9 parts of PEG 400-DS for the PGMS and 88 parts of water for water.

Example 47 - DDAC/9% PEG 400-DS/Mineral Spirits

The method of Example 46 is followed substituting 88 parts of mineral spirits for the water.

Example 48 - Didecyldimethylammonium hydroxide/PGMS/water

The method of Example 39 is followed substituting 3 parts of didecyldimethylammonium hydroxide for the didecyldimethylammonium chloride.

Example 49 - Didecyldimethylammonium carbonate/2.5% PGMS/water

The method of Example 39 is followed substituting 3 parts of didecyldimethylammonium carbonate for the didecyldimethylammonium chloride.

Example 50 - Didecyldimethylammonium carbonate/2.5% glycerol monolaurate (GML)/Water

The method of Example 49 is followed substituting 2.5 parts of GML for the PGMS.

Example 51 - Didecyldimethylammonium carbonate/2.5% glycerol monostearate (GMS)/Water

The method of Example 50 is followed substituting 2.5 parts of GMS for the GML.

Example 52 - Didecyldimethylammonium acetate/PGMS/water

The method of Example 39 is followed substituting 3 parts of didecyldimethylammonium acetate for the didecyldimethylammonium chloride.

Example 53 - Didecyldimethylammonium mixed coconut fatty acid carboxylate/PGMS/water

The method of Example 39 is followed substituting 5 parts of didecyldimethylammonium mixed coconut fatty acid carboxylate for the didecyldimethylammonium chloride, 5 parts of PGMS for the PGMS, and 90 parts of water for the water.

Example 54 - Didecyldimethylammonium chloride/PGMS/water

End grain pine wafers are weighed and then soaked with a waterproofer, wood preservative system prepared according to the method of Example 39 until the samples are saturated with the treating mixture. The samples are then air dried to constant weight to determine the uptake of the waterproofer, wood preservative system.

The treated wafers are removed, dried to constant weight, and weighed periodically to determine resistance to leaching.

The dried treated wafers are soaked in water for 30 minutes to determine swelling. Swell is measured as the increase in length of the sample compared to an untreated control, and the swell index for each is calculated.

Results are illustrated in Table 2 and Figures 1A and 1B.

Comparative Example 54A - Didecyldimethylammonium chloride

The method of Example 54 is followed substituting didecyldimethylammonium chloride for the waterproofer, wood preservative system.

Results are illustrated in Table 2 and Figures 1A and 1B.

Example 55 - DDAC/3% PGMS/Mineral Spirits

The method of Example 54 is followed substituting a waterproofer, wood preservative system prepared according to the method of Example 40 for the waterproofer, wood preservative system.

Results are illustrated in Table 2.

Example 56 - DDAC/6% PGMS/Mineral Spirits

The method of Example 54 is followed substituting a waterproofer, wood preservative system prepared according to the method of Example 41 for the waterproofer, wood preservative system.

Results are illustrated in Table 2.

Example 57 - DDAC/8% PGMS/Mineral Spirits

The method of Example 81 is followed substituting a waterproofer, wood preservative system prepared according to the method of Example 42 for the waterproofer, wood preservative system.

Results are illustrated in Table 2.

Example 58 - DDAC/12% PGMS/Mineral Spirits

The method of Example 54 is followed substituting a waterproofer, wood preservative system prepared according to the method of Example 43 for the waterproofer, wood preservative system.

Results are illustrated in Table 2.

Example 59 - DDAC/9% EGMS/Water

The method of Example 54 is followed substituting a waterproofer, wood preservative system prepared according to the method of Example 44 for the waterproofer, wood preservative system.
Results are illustrated in Table 2.

Example 60 - DDAC/10% EGDS/Water

The method of Example 54 is followed substituting a waterproofer, wood preservative system prepared according to the method of Example 45 for the waterproofer, wood preservative system.
Results are illustrated in Table 2.

Example 61 - DDAC/9% PEG 400-DS/Water

The method of Example 54 is followed substituting a waterproofer, wood preservative system prepared according to the method of Example 46 for the waterproofer, wood preservative system.
Results are illustrated in Table 2.

Example 62 - DDAC/9% PEG 400-DS/Mineral Spirits

The method of Example 54 is followed substituting a waterproofer, wood preservative system prepared according to the method of Example 47 for the waterproofer, wood preservative system.
Results are illustrated in Table 2.

Example 63 - Didecyldimethylammonium hydroxide/PGMS/water

The method of Example 54 is followed substituting a waterproofer, wood preservative system prepared according to the method of Example 48 for the waterproofer, wood preservative system.
Results are illustrated in Table 2 and Figures 1A and 1B.

Comparative Example 63A - Didecyldimethylammonium hydroxide

The method of Comparative Example 54A is followed substituting didecyldimethylammonium hydroxide for the didecyldimethylammonium chloride.
Results are illustrated in Table 2 and Figures 1A and 1B.

Example 64 - Didecyldimethylammonium carbonate/2.5% PGMS/water

The method of Example 54 is followed, substituting a waterproofer, wood preservative system prepared according to the method of Example 49 for the waterproofer, wood preservative system.
Results are illustrated in Table 2 and Figures 1A and 1B.

Comparative Example 64A - Didecyldimethylammonium carbonate

The method of Comparative Example 54A is followed substituting didecyldimethylammonium carbonate for the didecyldimethylammonium chloride to yield a clear solution.
Results are illustrated in Table 2 and Figures 1A and 1B.

Example 65 - Didecyldimethylammonium carbonate/2.5% GML/Water

The method of Example 64 is followed substituting 2.5 parts of GML for the PGMS.
Results are illustrated in Table 2.

Example 66 - Didecyldimethylammonium carbonate/2.5% GMS/Water

The method of Example 54 is followed substituting 2.5 parts of GMS for the PGMS.
Results are illustrated in Table 2.

Example 67 - Didecyldimethylammonium acetate/PGMS/water

The method of Example 54 is followed, substituting a waterproofer, wood preservative system prepared according to the method of Example 52, for the waterproofer, wood preservative system.
Results are illustrated in Table 2 and Figures 1A and 1B.

Comparative Example 67A - Didecyldimethylammonium acetate

The method of Comparative Example 54A is followed substituting didecyldimethylammonium acetate for the didecyldimethylammonium chloride.
Results are illustrated in Figures 1A and 1B.

Example 68 - Didecyldimethylammonium mixed coconut fatty acid carboxylate/PGMS/water

The method of Example 54 is followed substituting a waterproofer, wood preservation system prepared according to the method of Example 53 for the waterproofer, wood preservative system to yield an emulsion.
Results are illustrated in Table 2.

Comparative Example 68A - Didecyldimethylammonium mixed coconut fatty acid carboxylate

The method of Comparative Example 54A is followed substituting 5 parts of didecyldimethylammonium mixed coconut fatty acid carboxylate and 95 parts of water for the waterproofer, wood preservative system.
Results are illustrated in Table 2.

Example 69 - PGMS

The method of Example 54 is followed substituting a solution of 8 parts of PGMS and 92 parts of water for the waterproofer, wood preservative system.
Results are illustrated in Table 2.

Comparative Example 69A - Mineral spirits

The method of Example 54 is followed substituting a commercially available wax based biocide/mineral spirit based solution (Woodtreat MB* - KopCoat. Inc.)for the waterproofer, wood preservative system.
Results are illustrated in Table 2.
Table 2 illustrates the enhanced properties of waterproofer, wood preservative systems of the present invention.

## Properties of Waterproofer, Wood Preservative Systems

Table 2

| Example | 54 | 54A | 55 | 56 | 57 | 58 | 59 | 60 | 61 | 62 | 63 |
|---|---|---|---|---|---|---|---|---|---|---|---|
| Composition | | | | | | | | | | | |
| Quat | | | | | | | | | | | |
| Chloride | 3 | 100 | 3 | 3 | 3 | 3 | 3 | 3 | 3 | 3 | — |
| Hydroxy | — | — | — | — | — | — | — | — | — | — | 3 |
| Carbonate | — | — | — | — | — | — | — | — | — | — | — |
| Acetate | — | — | — | — | — | — | — | — | — | — | — |
| Mixed Coconut Fatty Acid Carboxylate | — | — | — | — | — | — | — | — | — | — | — |
| Waterproofer | | | | | | | | | | | |
| PGMS | 2.5 | — | 3 | 6 | 8 | 12 | — | — | — | — | 2.5 |
| EGMS | — | — | — | — | — | — | 9 | — | — | — | — |
| EGDS | — | — | — | — | — | — | — | 10 | — | — | — |
| PEG 400-DS | — | — | — | — | — | — | — | — | 9 | 9 | — |
| GML | — | — | — | — | — | — | — | — | — | — | — |
| GMS | — | — | — | — | — | — | — | — | — | — | — |
| Wax-Based in Mineral Spirits | — | — | — | — | — | — | — | — | — | — | — |
| Solvent | | | | | | | | | | | |
| Water | 94.5 | — | — | — | — | — | 88 | 87 | 88 | — | 94.5 |
| Mineral Spirits | — | — | 94 | 91 | 89 | 85 | — | — | — | 88 | — |
| Properties | | | | | | | | | | | |
| Swell Index (%) | 57 | — | 55 | 33 | 50 | 55 | 36 | 30 | — | — | 14 |
| Total Add On (%) | 5.4 | 35 | — | — | — | — | — | — | — | — | 4.8 |
| Solids or Add On Retained at 24 Hours Leaching, at Room Temperature (%) | — | — | — | — | — | — | — | — | — | — | — |
| Solids or Add On Retained at 300 Hours Leaching, at Room Temperature (%) | 3.2 | 0 | — | — | — | — | — | — | — | — | 3.5 |

## Properties of Waterproofer, Wood Preservative Systems

Table 2 (Continued)

| Example | 63A | 64 | 64A | 65 | 66 | 67 | 67A | 68 | 68A | 69 | 69A |
|---|---|---|---|---|---|---|---|---|---|---|---|
| Composition | | | | | | | | | | | |
| Quat | | | | | | | | | | | |
| Chloride | — | — | — | — | — | — | — | — | — | — | — |
| Hydroxy | 100 | — | — | — | — | — | — | — | — | — | — |
| Carbonate | — | 3 | 100 | 3 | 3 | — | — | — | — | — | — |
| Acetate | — | — | — | — | — | 3 | 100 | — | — | — | — |
| Mixed Coconut Fatty Acid Carboxylate | — | — | — | — | — | — | — | 5 | 5 | — | — |
| Waterproofer | | | | | | | | | | | |
| PGMS | — | 2.5 | | — | — | 2.5 | — | 5 | — | 8 | — |
| EGMS | — | — | — | — | — | — | — | — | — | — | — |
| EGDS | — | — | — | — | — | — | — | — | — | — | — |
| PEG 400-DS | — | — | — | — | — | — | — | — | — | — | — |
| GML | — | — | — | 2.5 | — | — | — | — | — | — | — |
| GMS | — | — | — | — | 2.5 | — | — | — | — | — | — |
| Wax-Based in Mineral Spirits | — | — | — | — | — | — | — | — | — | — | 1–2 |
| Solvent | | | | | | | | | | | |
| Water | — | 94.5 | — | 94.5 | 94.5 | 94.5 | — | 90 | 95 | 92 | — |
| Mineral Spirits | — | — | — | — | — | — | — | — | — | — | 98–99 |
| Properties | | | | | | | | | | | |
| Swell Index (%) | — | 71 | — | 14 | 57 | 57 | — | 50 | 14 | 0 | 43 |
| Total Add On (%) | 35 | 5.1 | 37 | 4.2 | 5.1 | 4.5 | 45 | 10 | 2.7 | 3.4 | 3.4 |
| Solids or Add On Retained at 24 Hours Leaching, at Room Temperature (%) | — | — | — | — | — | — | — | 103 | 103 | 100+ | 100+ |
| Solids or Add On Retained at 300 Hours Leaching, at Room Temperature (%) | 0 | 4.5 | 0 | 2.1 | 3.4 | 3 | 0 | — | — | — | — |

Many variations of the present invention will suggest themselves to those skilled in the art in light of the above detailed description. Such obvious variations are within the full intended scope of the appended claims.

### Claims

1. Use of a waterproofer composition for the treatment and preservation of wood, wherein said waterproofer composition comprises

(a) a compound having the formula

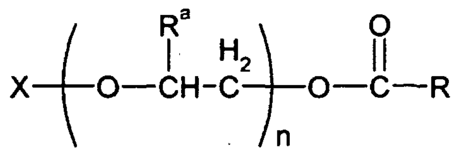

wherein: X is hydrogen or

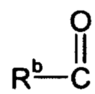 ,

R and $R^b$ independently are a (saturated or unsaturated) $C_9$-$C_{50}$ group,
$R^a$ is hydrogen or a methyl group; and
n is an integer from 1 to 10;

(b) a biocidal effective amount of at least one $C_1$-$C_{20}$ alkyl or aryl-substituted alkyl, $C_8$-$C_{20}$ alkyl quaternary ammonium composition; and

(c) optionally, a solvent,

said composition being metal-free.

**2.** Use of a waterproofer composition as defined in claim 1, wherein X is

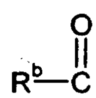 ,

R und $R^b$ each are a $C_{17}$ alkyl group, $R^a$ is a hydrogen, and n is 1.

**Patentansprüche**

**1.** Verwendung eines Hydrophobierungsmittels für die Behandlung und Konservierung von Holz, worin das Hydrophobierungsmittel

(a) eine Verbindung der Formel

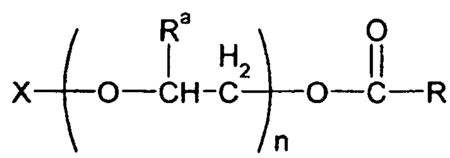

worin X Wasserstoff oder

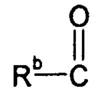

ist,
R und $R^b$ unabhängig voneinander eine (gesättigte oder ungesättigte) $C_9$-$C_{50}$-Gruppe sind,
$R^a$ Wasserstoff oder eine Methylgruppe ist und

n eine ganze Zahl von 1 bis 10 ist,
(b) eine biozid wirksame Menge wenigstens einer quartären $C_1$-$C_{20}$-Alkyl- oder arylsubstituierten Alkyl-$C_8$-$C_{20}$-alkylammoniumverbindung und
(c) gegebenenfalls ein Lösungsmittel

umfasst und metallfrei ist.

**2.** Verwendung eines Hydrophobierungsmittels nach Anspruch 1, worin X

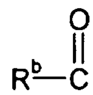

ist, R und $R^b$ beide eine $C_{17}$-Alkylgruppe sind, $R^a$ Wasserstoff ist und n 1 ist.

## Revendications

**1.** Utilisation d'une composition hydrophobisante pour le traitement et la préservation du bois, dans laquelle ladite composition hydrophobisante comprend

(a) un composé ayant la formule

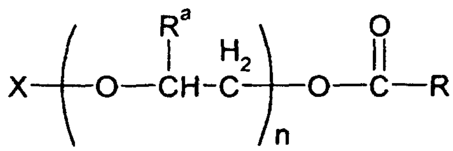

dans laquelle: X est l'hydrogène ou

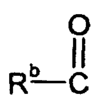 ;

R et $R^b$ sont indépendamment un groupement $C_9$-$C_{50}$ saturé ou insaturé ;
$R^a$ est l'hydrogène ou un groupement méthyl ; et
n est un nombre entier de 1 à 10 ;
(b) une quantité efficace d'un biocide d'au moins une composition d'ammonium quaternaire d'alkyle en $C_1$-$C_{20}$ ou d'alkyle en $C_1$-$C_{20}$ substitué par un aryle et d'alkyle en $C_8$-$C_{20}$ ; et
(c) optionnellement, un solvant,

ladite composition ne contenant aucun métal.

**2.** Utilisation d'une composition hydrophobisante selon la revendication 1, dans laquelle X est

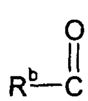 ,

R et $R^b$ sont tous deux un groupement alkyl en $C_{17}$, $R^a$ est un hydrogène, et n est 1.

FIG. 1A

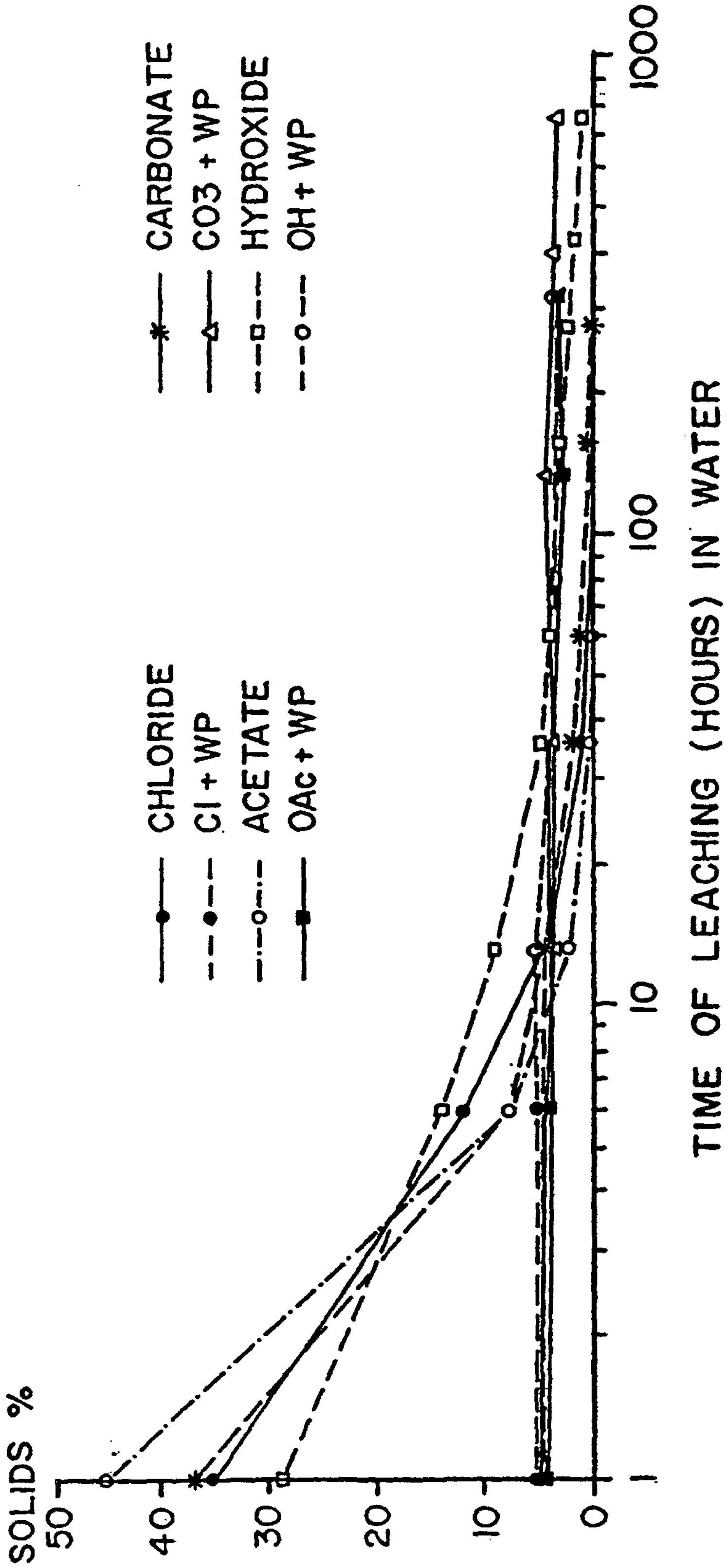
CHLORIDE
Cl + WP
ACETATE
OAc + WP
CARBONATE
CO3 + WP
HYDROXIDE
OH + WP
SOLIDS %
50
40
30
20
10
0
1
10
100
1000
TIME OF LEACHING (HOURS) IN WATER

FIG. 1B

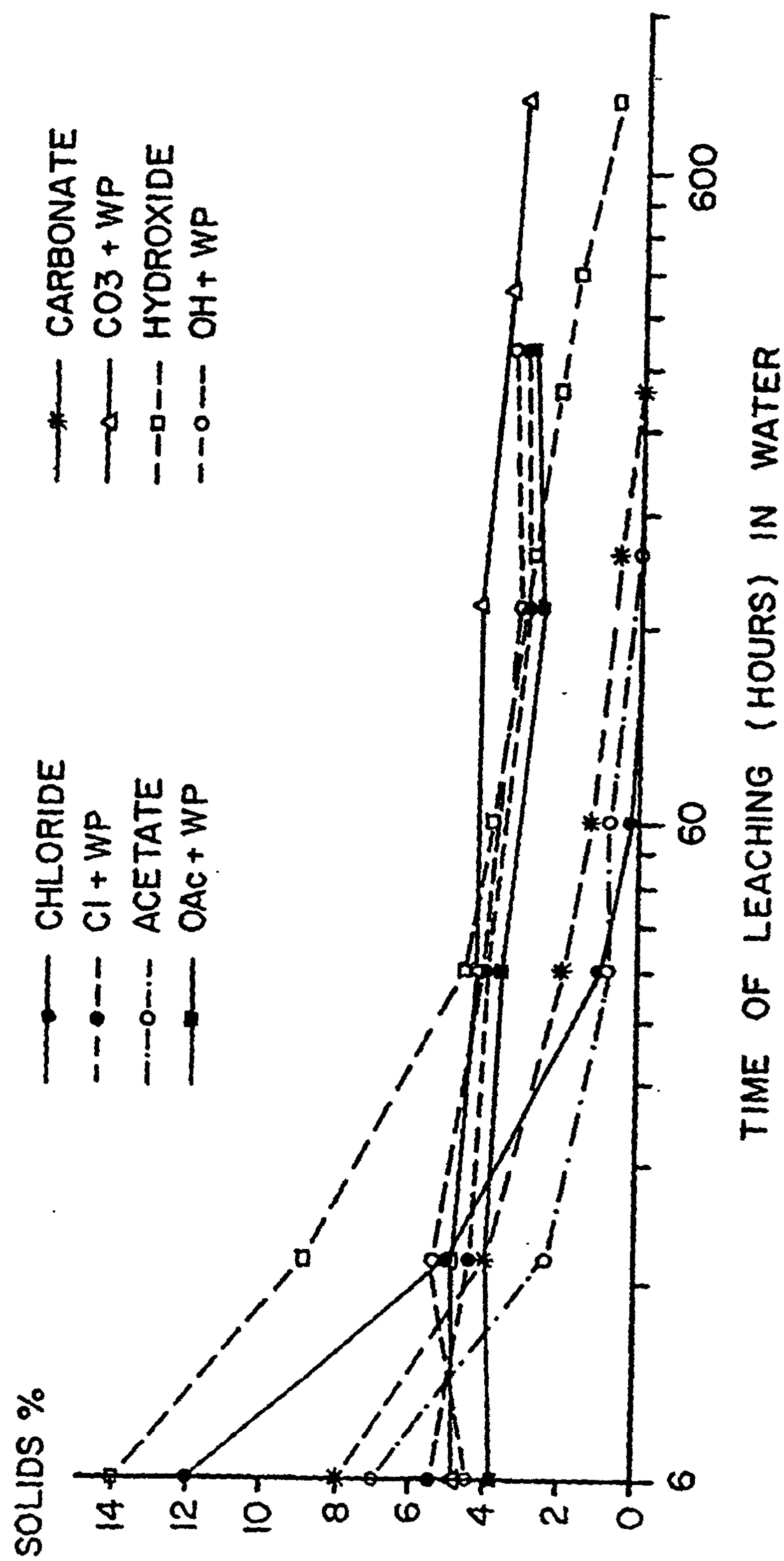
SOLIDS %
14
12
10
8
6
4
2
0
6
60
600
TIME OF LEACHING (HOURS) IN WATER
CHLORIDE
Cl + WP
ACETATE
OAc + WP
CARBONATE
CO3 + WP
HYDROXIDE
OH + WP